Europäisches Patentamt

European Patent Office

Office européen des brevets

(19)

(11) Veröffentlichungsnummer: **0 022 240**

**A1**

(12) **EUROPÄISCHE PATENTANMELDUNG**

(21) Anmeldenummer: **80103726.8**

(22) Anmeldetag: **01.07.80**

(51) Int. Cl.³: **G 01 N 33/72**

(30) Priorität: **04.07.79 DE 2926980**

(43) Veröffentlichungstag der Anmeldung:
**14.01.81 Patentblatt 81/2**

(84) Benannte Vertragsstaaten:
**BE DE FR GB IT NL**

(71) Anmelder: **BEHRINGWERKE AKTIENGESELLSCHAFT**
**Postfach 1140**
**D-3550 Marburg/Lahn(DE)**

(72) Erfinder: **Kohl, Helmut, Dr.**
**Goethe-Strasse 32**
**D-3552 Wetter(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) **Mittel zum Nachweis von Bilirubin.**

(57) Es wird ein Mittel zum Nachweis von Bilirubin im Urin beschrieben, das aus einem saugfähigen Trägermaterial, einer Diazoniumverbindung, die mit Bilirubin unter Farbänderung reagiert, einem Puffersalz und einem kationischen Netzmittel besteht.

EP 0 022 240 A1

BEHRINGWERKE AKTIENGESELLSCHAFT    Hoe 79/B 010 Dr.HA/Bl    .


Mittel zum Nachweis von Bilirubin
_____

Die Erfindung betrifft ein Mittel zum Nachweis von Bilirubin im Urin, bestehend aus einem saugfähigen Trägermaterial, einer Diazoniumverbindung, die mit Bilirubin
unter Farbänderung reagiert, einem Puffersalz und einem
Netzmittel.

Zur Beschleunigung der Reaktion von Bilirubin mit
Diazoniumverbindungen in Lösung wurden bereits anionische  (F.Watanabe et al., Clin.Chim.Acta 88 (1978),149)
oder nichtionische Netzmittel (DOS 2 110 658, Schweizer
Patent 512 072) beschrieben.

Ebenso wurden anionische und nichtionische Netzmittel
für die Herstellung von Mitteln zum Nachweis von Bilirubin verwendet, in denen das Reaktionssystem auf ein
Trägermaterial aufgebracht ist.  Solche Mittel sind als
"Teststreifen" bekannt.

Von Nachteil ist aber bei solchen entsprechend dem Stand
der Technik hergestellten Teststreifen, daß sie nur eine
schwache Farbintensität entwickeln, selbst bei quantitativem Umsatz.  Dieser Nachteil, der besonders die Aussage im entscheidenden physiologisch/pathologischen
Bereich unsicher oder unmöglich macht, kann durch die
erfindungsgemäße Verwendung eines kationischen Netzmittels als Aktivator behoben werden.

Gegenstand der Erfindung ist demnach ein Mittel zum Nachweis von Bilirubin im Urin, bestehend aus einem saugfähigen Trägermaterial, einer Diazoniumverbindung, die mit Bilirubin unter Farbänderung reagiert, einem Puffersalz und einem Netzmittel, dadurch gekennzeichnet, daß das Netzmittel ein kationisches Netzmittel ist oder ein solches enthält.

Durch Zugabe von kationischen Netzmitteln, besonders aber quartären Ammoniumsalzen wie beispielsweise N-Äthyl-N,N-dimethyl-N-dodecylammoniumhalogeniden, zur Tränklösung wird die Farbintensität so verstärkt, daß beispielsweise bei Verwendung von Halogenbenzol-diazoniumsalzen Bilirubin schon im physiologischen Bereich($< 0,5$ mg/dl) durch eine kräftige Farbentwicklung angezeigt wird. Die Netzmittel werden in einer Konzentration von 0,1 - 10 Vol.-%, vorzugsweise 1 - 3 Vol.-% verwendet.

Beispiel

In 100 ml Wasser oder einer Mischung aus Wasser und einem polaren Lösungsmittel werden nacheinander gelöst :

1 g    m-Phosphorsäure,

5 g    Weinsäure

1 g    N-Ethyl-N,N-dimethyl-N-dodecylammoniumbromid und

1 g    2,4-Dichlorbenzoldiazoniumfluoroborat.

Filterpapier wird mit dieser Lösung imprägniert und bei $40°C$ getrocknet. Durch Eintauchen des imprägnierten Filterpapiers in eine bilirubinhaltige Lösung, beispielsweise bilirubinhaltigen Urin, entsteht entsprechend der Bilirubinkonzentration eine rot-braune bis intensiv rote Färbung. Die Nachweisgrenze liegt bei 0,5 mg/dl Bilirubin

Durch die Variation der Menge des Aktivators kann die Nachweisgrenze auf 0,2 mg % Bilirubin gesenkt werden. Zweckmäßigerweise wird man aber eine Menge wählen, bei der physiologische Bilirubinmengen noch nicht angezeigt werden.

Das im Beispiel eingesetzte Netzmittel kann durch eines der nachfolgend aufgeführten Netzmittel mit ähnlichem Ergebnis ersetzt werden :

Tetraethylammoniumbromid
Tetramethylammoniumbromid
N-Benzyl-N,N-dimethyl-N-dodecylammoniumbromid
N-Ethyl-N,N-dimethyl-N-hexadecylammoniumbromid
N,N,N-Trimethyl-hexadecylammoniumbromid
N-Benzyl-N,N,N-trimethylammoniumbromid
N-Tetradecyl-N,N-dimethylbenzyl-ammoniumchlorid
Tetrabutyl-ammoniumhydrogensulfat
Hexadecyl-trimethylammoniumbromid
Octadecenylamin

Patentansprüche:

1.  Mittel zum Nachweis von Bilirubin, bestehend aus einem saugfähigen Trägermaterial, einer Diazoniumverbindung, einem Puffersalz und einem Netzmittel, dadurch gekennzeichnet, daß das Netzmittel ein kationisches Netzmittel ist oder ein solches enthält.

2.  Mittel entsprechend Anspruch 1, dadurch gekennzeichnet, daß das Netzmittel ein quartäres Ammoniumsalz ist oder ein solches enthält.

3.  Mittel entsprechend Anspruch 1, dadurch gekennzeichnet, daß das Netzmittel N-Ethyl-N,N-dimethyl-N-dodecylammoniumhalogenid ist oder dieses enthält.

Europäisches
Patentamt

**EUROPÄISCHER RECHERCHENBERICHT**

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | <u>DE - A - 2 240 471</u> (BOEHRINGER MANNHEIM) <br> * Ansprüche 1 bis 4; Seite 5, Zeilen 19 bis 25 * <br><br> -- <br><br> Chemical Abstracts Band 89, Nr. 26 25. Dezember 1978 Columbus, Ohio, USA C. SAUTEREY " Effect of charge on the solubilizing power and structure of anionic tensioactive microemulsions" Seite 380, rechte Spalte, Abstract Nr. 221325z * die letzten 2 Zeilen * & C.R. Hebd. Seances Acad. Sci., Ser., C, Band 287, Nr. 4, 1978 Seiten 77 bis 80 <br><br> -- <br><br> <u>DE - B - 2 110 658</u> (BOEHRINGER MANNHEIM) * ganze Druckschrift * <br><br> ---- | 1 <br><br><br><br><br><br> 2,3 | G 01 N 33/72 <br><br><br><br> **RECHERCHIERTE SACHGEBIETE (Int. Cl.³)** <br><br> G 01 N 33/72 |

Kategorie: A

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. | | |
|---|---|---|---|
| Recherchenort <br> Berlin | Abschlußdatum der Recherche <br> 08-09-1980 | Prüfer <br> SCHWARTZ | |

EPA form 1503.1   06.78